# EUROPEAN PATENT APPLICATION

(11) **EP 4 636 777 A1**
(43) Date of publication of application: **22.10.2025**
(21) Application number: 24170983.1
(22) Date of filing: 18.04.2024
(51) Int. Cl.: G16H 20/40, G16H 30/00, G16H 30/40, A61B 6/00, A61B 6/46

(54) **COMPUTER-IMPLEMENTED METHOD FOR OPERATING A MEDICAL IMAGING DEVICE, MEDICAL IMAGING DEVICE, COMPUTER PROGRAM, AND ELECTRONICALLY READABLE STORAGE MEDIUM**

(71) Applicant: Siemens Healthineers AG, 91301 Forchheim (DE); Herlev and Gentofte Hospital, 2730 Herlev (DK)
(72) Inventor: Dr. Taubmann, Oliver, 91365 Weilersbach (DE); Dr. Sühling, Michael, 91052 Erlangen (DE); Dr. Müller, Christoph Felix, 2200 Copenhagen (DK); Paaske, Adam William Augustinus, 6000 Kolding (DK)
(74) Representative: Siemens Healthineers Patent Attorneys

(57) **Abstract**

The invention relates to a computer-implemented method for operating a medical imaging device (14), wherein, for preparing an examination scan of a subject, a preparation image (6) of an acquisition region (5) is acquired, the preparation image (6) being defined with respect to a first coordinate system and used to define at least one parameter for acquiring an examination image, comprising the steps of
- retrieving, from a storage (2) comprising at least one prior imaging result data set (1) of the subject, a source data set chosen from the at least one prior imaging result data set (1), each prior imaging result data set (1) comprising a prior image showing at least a part of the acquisition region (5) and annotation information indicating at least one feature of interest (9) of the subject, wherein the annotation information comprises a location information describing a location of a respective feature of interest (9) in a second coordinate system of the prior image,
- registering the prior image of the source data set and the preparation image (6) to obtain registration information comprising a transformation from the second coordinate system to the first coordinate system,
- compiling an output image (3, 12) comprising the preparation image (6) and at least one visual representation (8), which is positioned correctly according to the location information of the source data set for at least one direction, of all features of interest (9) within a display region (4) comprising the acquisition region (5), and
- outputting the output image (3, 12) at a display device.

## Description

The invention concerns a computer-implemented method for operating a medical imaging device, wherein, for preparing an examination scan of a subject, a preparation image of an acquisition region is acquired, the preparation image being defined with respect to a first coordinate system and used to define at least one parameter for acquiring an examination image. The invention further concerns a medical imaging device, a computer program, and an electronically readable storage medium.

In medical imaging, it is known to acquire preparation images. Based on such a preparation image, acquisition parameters for the acquisition of at least one examination image may be defined. For example, the preparation image may provide an overview over the anatomy of the subject, in particular a patient, to choose the field of view such that it comprises features of interest, for example certain anatomical structures like organs or lesions. In computed tomography (CT), a topogram is often required as preparation image. A topogram is a two-dimensional x-ray projection image acquired with the computed tomography device and used by the operator to (manually and/or automatically) define the scan range, in particular in the axial direction, and/or the field of view for reconstruction. The topogram may also be used to identify (manually and/or automatically) potential issues that may impede diagnostic image quality, for example the presence of metal objects. Appropriate countermeasures may be taken. In magnetic resonance imaging (MRI), localizers can be acquired as preparation images.

Medical imaging is routinely used to diagnose diseases, such that they can be treated, and to monitor the progress of therapy. Often, so-called follow-up examination scans of subjects, in particular patients, are performed. These examination scans may, for example, serve to monitor the progression or healing of medical conditions. For example, a patient diagnosed with cancer may undergo CT scans several times over the course of the therapy to monitor the response of the disease. In order to plan an examination scan, it is important to know the location and extent of the internal feature of interest to be examined.

In the state of the art, to ensure that all relevant features are inside the planned scan range and/or field of view and are reconstructed at sufficient resolution, the user relies on communication with a referring physician and/or radiologist to learn of any subject-specific requirements and/or needs to manually check prior scans/findings in a different system, for example a picture archiving and communication system (PACS) or an electronic patient record.

This solution is prone to errors, since there may be miscommunication (or even a lack of communication regarding a specific aspect) between the radiologist/clinician and the user of the medical imaging device (the radiographer), and/or the user does not have the capacity, in particular regarding time and expertise, to manually check prior scans or check them diligently enough. In this case, an examination scan may have to be repeated. Even if no errors are made, the need for additional communication or manual search for and checking of prior scans introduces inefficiencies in the workflow.

It is an object of the invention to provide improved support to users of medical imaging devices regarding the planning of the acquisition of an examination image, in particular such that at least one feature of interest is imaged.

This object is achieved by providing a computer-implemented method, a medical imaging device, a computer program and an electronically readable storage medium according to the independent claims. Preferred embodiments are described by the dependent claims.

According to the invention, a method as initially described comprises the steps of:
- retrieving, from a storage comprising at least one prior imaging result data set of the subject, a source data set chosen from the at least one prior imaging result data set, each prior imaging result data set comprising a prior image showing at least a part of the acquisition region and annotation information indicating at least one feature of interest of the subject, wherein the annotation information comprises a location information describing a location of a respective feature of interest in a second coordinate system of the prior image,
- registering the prior image of the source data set and the preparation image to obtain registration information comprising a transformation from the second coordinate system to the first coordinate system,
- compiling an output image comprising the preparation image and at least one visual representation, which is positioned correctly according to the location information of the source data set for at least one direction, of all features of interest within a display region comprising the acquisition region, and
- outputting the output image at a display device.

It is hence proposed to automatically identify suitable prior images, register them to the preparation image of the current examination scan, and subsequently display, in particular in a user interface showing the preparation image, any relevant features of interest, for example prior findings, determined in prior scans of the subject. As a result of the registration, the at least one feature of interest is displayed at the correct location regarding the preparation image. In particular, visual representations of the features of interest may be overlaid onto the preparation image or positioned relative to the preparation image in the display region at the correct position in at least one direction, since the transformation can be used to transfer the location information from the second coordinate system to the first coordinate system. In other words, the output image is generated for the display region in the second coordinate system using the preparation image, the annotation information of the source dataset and the transformation, which is applied to the location information. Key components of the method hence comprise registration of a prior scan to the preparation image of the current examination scan and the subsequent display of prior findings with the preparation image based on the registration result. It is applied to stand-alone examination scans, meaning the prior imaging result data sets relate to prior scans a longer time ago, for example at least one month ago.

The output image may be part of a user interface, wherein the user can choose and/or adapt and/or confirm acquisition parameters, in particular acquisition parameters defining the field of view for acquisition and/or reconstruction of the examination image. The information provided in the output image can hence be used to choose improved acquisition parameters, in particular ensure that the at least one feature of interest is in the field of view and/or depicted in sufficiently high quality.

The herein presented method has advantages regarding multiple aspects of the method. Prefetching of a suitable prior image and its registration to the preparation image circumvent the need for manual search and visual comparison by the user. By being able to automatically display the features of interest, in particular prior findings, directly in the preparation image view, no additional manual efforts are required, and the likelihood of mistakes is reduced. Hence, medical imaging devices become smarter and more user-friendly. In particular, follow-up examinations (which account for a very large portion of all examination scans), for example regarding CT scans of oncology patients, may be carried out more quickly and with a reduced probability for errors.

Hence, preferably, the examination scan is a follow-up examination scan, wherein the at least one prior imaging result data set relates to a previous examination scan and/or is a baseline scan and/or wherein the examination scan is performed for comparison with the at least one prior imaging result data set. For example, the prior image and annotation information may relate to a prior scan, which has been acquired a certain time interval before the current scan, for example at least one month ago. The examination scan may be part of a series of scans, in particular at regular or irregular intervals, to monitor how well a treatment works, a wound heals and/or how a medical condition evolves. In particular, the examination image or results of an analysis of the examination image may be compared to the prior image and/or the annotation information or other previous analysis results. The source data set and/or further prior imaging result data sets may hence also be used regarding analysis of the examination image, in particular regarding at least one of the at least one feature of interest. For example, the growth or other evolution of a lesion can be evaluated.

The method may be applied to any imaging modality and/or process using a preparation image, for example localizers of medical resonance imaging. Preferably, however, the medical imaging device is a computed tomography device, and the preparation image is a topogram. Computed tomography relies on x-rays, such that the method may advantageously also result in less radiation to be applied to the subject, in particular patient.

Regarding CT, the at least one direction may comprise at least an axial direction, which corresponds to the superior-inferior direction of the patient. That is, relative positioning of the subject regarding the acquisition of the examination image is most important along the rotational axis of the acquisition arrangement (x-ray source and x-ray detector) of the medical imaging device, which also is the axial direction of the gantry. It usually also corresponds to the longitudinal direction of the patient table. By providing information regarding the axial position of the at least one feature of interest, the radiation may be directed and confined to the correct and required part of the body.

While, in principle, the source data set may be chosen manually, it is preferred to provide automatic selection of the source data set. Hence, preferably, the source data set is chosen from the multiple prior imaging result data sets using at least one selection function, wherein the selection function evaluates at least one selection criterion. Here, at least one of the at least one selection criterion may be chosen from the group comprising
- the acquisition date of the prior imaging result data set, wherein more recent prior imaging result data sets are preferred,
- the body region the prior imaging result data set relates to, wherein prior imaging result data sets relating to the same body region as the examination scan are preferred,
- the modality the prior imaging result data set has been acquired with, wherein prior imaging result data sets being acquired with the same (or at least a similar) modality as the one used for the examination scan are preferred,
- motion state information associated with the prior image, wherein prior images having a similar motion state to the preparation image are preferred, and
- acquisition protocol parameters, wherein prior imaging result data sets using acquisition protocols similar to that of the acquisition scan are preferred.

Selection and automated provision of the source data set at the medical imaging device may take multiple selection criteria into account. For example, advanced selection functions for identifying suitable prior scans, in particular employing machine learning/artificial intelligence, which have already been proposed in the state-of-the-art in other contexts, may also be applied in the current invention. Hence, a trained function may be used as the selection function.

Generally, prior images being acquired with the same modality and showing the same body region may be preferred, since registration is facilitated. In particular, furthermore, similar acquisition protocols also simplify registration. For example, similarity scores may be determined and used. In embodiments, motion state information may be associated with the prior image, describing at least one motion state, in which the prior image or a respective sub-image has been acquired, the motion state relating to breathing motion and/or heart motion. Here, similar motion states are preferred, further simplifying the registration process. However, a main and important selection criterion is the acquisition date, in particular regarding features of interest, for example in regard of monitoring their evolution. If multiple selection criteria are used, they may be weighted differently, for example assigning high weight to the acquisition date and the body region and lower weights to other selection area.

However, in a simple heuristic approach sufficient for many applications, the prior imaging result data set relating to the latest scan of the body region under examination, in particular the acquisition region or the display region, may be chosen as the source data set.

In embodiments, the at least one prior imaging result data set may be retrieved from an electronic patient record (often also electronic health record - EHR). In preferred embodiments, all prior imaging result data sets in the electronic patient record may be stored using a common second coordinate system. In such a case, prior images may be used for registration irrespective of acquisition date and up-to-dateness. For example, a prior imaging result data set for use as the source data set can be compiled comprising a prior image which is optimal for registration and the most current annotation information regarding the body region subject to examination.

The source data said can automatically be prefetched, that is retrieved, from a storage location external to the medical imaging device, for example from a picture archiving and communication system (PACS).

In embodiments, the prior image may be a preparation image of a prior examination scan using the same modality, simplifying the registration. This may hence provide an incentive to also store preparation scans in long-term storages like PACS. For example, both the preparation image and the prior image may be topograms, allowing easy and robust 2D-2D registration.

In other preferred embodiments, the preparation image is two-dimensional, and the prior image is at least three-dimensional, such that registration is performed as 2D-3D-registration. For example, a topogram may be registered to a three-dimensional prior image, in particular a prior computed tomography volume and/or stack of sectional images/slice images. 2D-3D-registration algorithms and functions have already been proposed in the state of the art. In preferred embodiments, the 2D-3D-registration comprises at least one of
- landmark-based registration, and
- minimizing a difference metric between preparation image and a two-dimensional reference image determined, in particular by forward projection, from the prior image, in particular based on an assumed acquisition geometry of the preparation image in the second coordinate system.

For example, known landmarks of corresponding anatomical structures/locations may be automatically detected in both the preparation image and the prior image. Thereafter, as the transformation, a suitable geometric transformation (for example a homography) that maps the 3D landmark positions to their respective 2D landmark positions, in particular projected positions in the case of a topogram, may be determined. Additionally or alternatively, a difference metric between the preparation image and reference image can be minimized. For example in the case of a topogram, the reference image may be a virtual (simulated/computed) X-ray projection image (DRR, "digitally reconstructed radiograph") from the 3D prior image, projected according to an estimate of the projection geometry (as acquisition geometry) that is refined during the optimization process. Generally speaking, the assumed acquisition geometry may also be optimized during registration, in particular the optimization process.

In particular regarding body regions which are subject to physiological motion, in particular breathing motion and/or heart motion, like abdomen or thorax, the motion state may also be taken into account. As discussed above, motion state information may be associated with the prior image, describing at least one motion state, in which the prior image or a respective sub-image has been acquired, the motion state relating to breathing motion and/or heart motion. A suitable three-dimensional prior image can be selected (as already discussed above) and/or a suitable three-dimensional sub-image of the four-dimensional prior image can be chosen for registration based on the motion state information.

However, other effects may also impede registration quality. For example, the size and/or position of inner organs may change over time, preventing perfect registration, which, however, is not required in this application. In particular, it may be sufficient to use rigid registration.

In preferred embodiments, the preparation image may be a two-dimensional projection image, in particular a topogram, and the annotation information may comprise a three-dimensional location information, wherein, during compilation of the output image, the three-dimensional location information is forward projected into the projection geometry of the preparation image according to the transformation to facilitate correct positioning of the respective feature in the output image. Hence, any (relevant) features, in particular findings, of the prior scan of the source data set may be projected using the projection geometry of the preparation image to be displayed in the anatomically corresponding position in the preparation image view, that is, the output image.

In especially preferred embodiments, if a feature lies outside the acquisition region according to the transformation, a warning may be output to a user and/or the feature may be displayed at the correct position according to the location information outside the preparation image in the display region. For example, if a feature of interest lies outside the acquisition region, a notice can be displayed to the user, informing him of this shortcoming. For example, the user may then consider acquiring another preparation image with an adapted field of view, for example, in the case of topogram, an adapted axial scan range, wherein the respective feature of interest is comprised in the new, adapted field of view. To provide further information, in particular regarding adaptation of the field of view for a further preparation image and/or regarding manual and/or automatic adjustment of the field of view of the examination image, in preferred embodiments, wherein the display region is larger than the acquisition region or can be suitably chosen, the location of the feature of interest outside the acquisition region, at least in the axial direction, can be correctly displayed in the display region, in particular to scale. Hence, the user can easily determine required adaptations of the field of view.

Preferably, at least one acquisition parameter for the examination image, which describes the field of view of the examination image, in particular an axial examination range, is automatically adapted such that the feature lying outside the acquisition region is included in the field of view of the acquisition image. Hence, for example, proposals for adapted acquisition parameters may be automatically determined, in particular such that the feature of interest is included in the field of view of the acquisition image, and may be presented to the user for confirmation. In this manner, further support for the user is provided.

It is noted that acquisition parameters of the examination image, in particular as proposals to be confirmed by the user, may also be determined automatically for other use cases, for example regarding desired quality of depiction of the feature of interest in the examination image. Preferably, such a proposal is at least partly also based on annotation information, for example the type of feature.

Preferably, an expected location error in at least one direction, in particular the superior-inferior and/or axial direction, due to breathing and/or registration is determined and visualized in the output image by at least one visualization element. In this manner, potential misalignment due to organ movement and/or registration errors can be accounted for. For example, a margin of error could be added to the visual representation of the feature of interest, in particular its location in at least one direction. If, for example, an axial dimension of the feature of interest, for example by marking an axial range, is indicated in the output image, extensions to this visual representation/display element of the visual representation may be added. For example, in the thorax or abdomen area, typical values for superior-inferior displacement due to breathing, which is the main contributor of misalignment, may be used. Expected breathing motion may be taken from a database of empirically determined values, in particular associated with body regions and/or anatomical structures, and/or may be determined based on patient-specific prior information, which may, for example, be comprised in the source data set and/or at least one other prior imaging result data set, for example associated with a four-dimensional prior image. Such 4D scans are, for example, known from radiation treatments. Additional information regarding potential misalignment due to breathing allow to choose the field of view of the examination image to reliably acquire image data from the corresponding feature of interest. If, in less preferred embodiments, registration errors are taken into account, these could be provided by used registration functions.

Features of interest, in particular prior findings, may be provided in prior imaging result data sets in different forms. For example, the annotation information may comprise at least one chosen from the group of
- DICOM meta information,
- a reference to a partial image of the or a prior image, in particular to a slice,
- at least one extension of the feature, in particular a lesion diameter,
- a textual entry describing the position of the feature in at least one of the at least one direction, and
- an, in particular three-dimensional, segmentation result of the feature.

In concrete embodiments, often used simple annotation information, like diameters of a target lesion in a single slice or sectional image or references to slice numbers, can already be used to roughly determine the location of the feature of interest in at least one direction, for example the axial direction (which usually also is the stacking direction of sectional/slice images). Furthermore, textual information, for example diagnosis comments, may also be analysed as location information. In preferred, ideal cases, the annotation information may comprise a full three-dimensional, automatic or semi-automatic segmentation of the feature of interest. Annotation information may be stored in prior images as DICOM meta information, using well-known standard.

The invention further concerns a medical imaging device, in particular computed tomography device, for an examination scan of a subject, comprising an acquisition arrangement, a display device and a control device, wherein the control device comprises:
- an acquisition unit for controlling the acquisition arrangement to acquire a preparation image of an acquisition region, the preparation image being defined with respect to a first coordinate system and used to define at least one parameter for acquiring an examination image, and the examination image,
- a storage interface for retrieving, from a storage comprising at least one prior imaging result data set of the subject, a source data set chosen from the at least one prior imaging result data set, each prior imaging result data set comprising a prior image showing at least a part of the acquisition region and annotation information indicating at least one feature of interest of the patient, wherein the annotation information comprises a location information describing a location of a respective feature of interest in a second coordinate system of the prior image,
- a registration unit for registering the prior image of the source data set and the preparation image to obtain registration information comprising a transformation from the second coordinate system to the first coordinate system,
- a compilation unit for compiling an output image comprising the preparation image and at least one visual representation, which is positioned correctly according to the location information of the source data set for at least one direction, of all features of interest within a display region comprising the acquisition region, and
- an output unit for outputting the output image at the display device.

The control device may comprise at least one processor and at least one storage means. It may be connected, in particular via a communication link, to the storage, wherein the prior imaging result data sets are stored. Functional units may be implemented by software and/or hardware to perform steps of a method according to the invention. Acquisition units for controlling the acquisition arrangement to acquire image data are already known. A reconstruction unit may be associated with the acquisition unit or integrated into the acquisition unit for reconstructing images from raw data. In addition to the registration unit, the compilation unit and the output unit, which may generally be responsible for providing a user interface, additional functional units may be provided to perform steps of additional embodiments of the invention. For example, the control device may also comprise a selection unit for selecting the source data set from multiple prior imaging result data sets.

A computer program according to the invention comprises program means such that, when the computer program is executed on a control device of a medical imaging device, the control device is caused to execute the steps of a method according to the invention. The computer program may be stored on an electronically readable storage medium according to the invention, which thus comprises control information comprising at least one computer program according to the invention, such that, when the storage medium is used in a control device of a medical imaging device, the control device is configured to perform a method according to the invention. The storage medium may preferably be a non-transient storage medium, for example a CD-ROM.

Other objects and features of the present invention will become apparent from the following detailed description considered in conjunction with the accompanying drawings. The drawings, however, are only principal sketches designed solely for the purpose of illustration and do not limit the invention. The drawings show:
Fig. 1 a flowchart of an embodiment of a method according to the invention,
Fig. 2 schematically a first output image,
Fig. 3 schematically a second output image,
Fig. 4 a medical imaging device according to the invention, and
Fig. 5 the functional structure of the control device of the medical imaging device of Fig. 4.

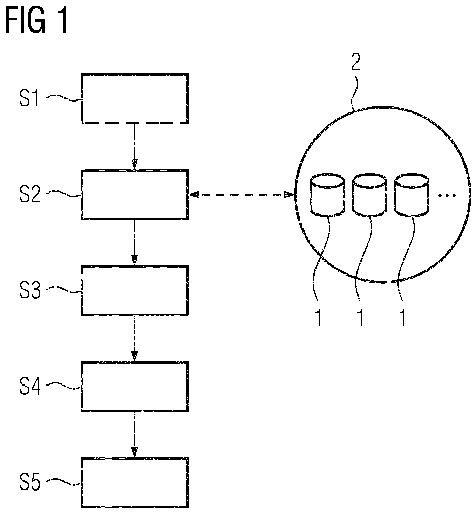

Fig. 1 shows a flowchart of a general embodiment of a method according to the invention. The embodiment exemplarily relates to an examination scan of a patient, performed with a computed tomography device as medical imaging device. Generally, in the examination scan, in a step S1, a topogram covering an axial region of the patient (that is, a body region in the superior-inferior direction, which corresponds to the axial direction of a gantry of the computed tomography device and hence the direction of the rotational axis of the acquisition arrangement) is acquired as a preparation image. The topogram is a two-dimensional projection image defined in a first coordinate system used by the medical imaging device. As other preparation images, for example localizers in MRI, the topogram is used to defined acquisition parameters for the main scan to acquire an examination image (as a computed tomography image reconstructed from multiple projections) of the patient. In particular, the topogram is used to ensure that all regions of interest are included in the field of view of the examination image. Hence, the topogram (or generally, the preparation image) is usually shown in a user interface on a display device of or associated with the medical imaging device. In the method described here, this view is extended by adding visual representations of features of interest, in particular prior findings.

Therefore, in a step S2, a source data set is selected from multiple prior imaging result data sets 1 in a storage 2 and retrieved from the storage 2. Each prior imaging result data set 1 comprises a prior image and annotation information regarding at least one feature of interest and its location with regard to a second coordinate system, in which the prior image is defined. For example, the prior image can be stored in a DICOM format, and the annotation information may be, at least partly, included as meta data. The location information included in the annotation information may be a rough description of the location of the feature of interest in at least one direction, for example a slice number in a slice stack or a textual description, but is preferably more precise. For example, the annotation information may comprise a, preferably 3D, segmentation result and/or a measurement result regarding at least one extension of the feature. Features of interest are, preferably, prior findings, for example lesions.

In this example, the examination scan is a follow-up examination scan, for example for monitoring the progress of a treatment/a therapy and/or the development of a disease, for example a tumor or other medical condition. In this sense, the examination scan may be understood as a stand-alone scan, meaning the prior scans described by the prior imaging result data sets 1 were acquired with a temporal distance, for example at least one month ago.

For selecting a suitable prior imaging result data set 1 as source data set, selection criteria may be evaluated, in particular in a weighed manner. Such selection criteria may comprise the date of acquisition of the prior image, the body region examined, the acquisition protocol used, the state of motion shown in the prior image regarding breathing and/or heart motion, and the imaging modality used. The more similar the prior image is to the preparation image, the easier the registration, however, proximity in time is also important, in particular regarding planned comparisons of the examination image and/or its annotations with prior scan results.

In an easily implementable approach, that is likely to suffice in most scenarios, the prior imaging result data set 1 relating to the latest scan of the same body region in the patient is selected as the source data set. In other approaches, however, more complex selection functions, in particular trained selection functions, may also be employed in step S2.

The storage 2 may be a PACS. In embodiments, the prior imaging result data sets 1 may be stored in an electronic patient record of the patient. They may preferably all use the same second coordinate system, allowing to compile and select prior imaging result data sets 1 comprising a prior image optimal for registration to the preparation image and the latest annotation information regarding relevant features of interest.

In a step S3, the prior image of the source data set and the preparation image, in this case the topogram, are co-registered to determine a transformation from the second coordinate system to the first coordinate system. In other words, the coordinate systems need to be aligned to establish spatial correspondence such that features of interest identified in the prior scan can be displayed at the corresponding anatomical position in the preparation image.

In some embodiments, the preparation image may also be a topogram, preferably using the same projection plane (for example a coronal plane). The prior image may even have been acquired with the same or a comparable medical imaging device, for example in a prior scan of a series of examination scans relating to a certain feature of interest. In such a case, 2D-2D registration may be performed in step S3.

However, in many cases, the prior image may be a three-dimensional image, for example a reconstructed computed tomography or magnetic resonance volume and/or slice stack. 2D-3D registration is performed. Exemplary concrete approaches comprise landmark-based methods and approaches trying to minimize a difference metric regarding the respective image information. In the topogram embodiment discussed here, a projection geometry may be assumed, and a reference image may be determined by forward projecting from the three-dimensional prior image. Such a forward projected reference image is also known as DRR (digitally reconstructed radiograph) and may be determined by simulation or direct computation. A difference metric between the reference image and the preparation image is minimized, also refining the acquisition geometry in the optimization process.

It is noted that perfect registration may not be possible in every case. While, when the prior image is four-dimensional covering a full breathing and/or heart cycle, a suitable sub-image showing the same motion state may be selected for registration to reduce deviations due to breathing and/or heart motion, such motion, in particular breathing motion, may also be taken into account when preparing an output image for display, as further discussed below. Perfect registration, however, is no prerequisite for the support option for the user provided here.

In a step S4, an output image is determined from the preparation image, the annotation information and the transformation. The output image is output in step S5 to a user as part of a user interface. Given the registration performed in step S3, any features of interest annotated/detected in the prior scan of the source data set can be added to the output image, in particular, for the topogram, projected with the same projection geometry, to be displayed in the anatomically corresponding position in the display region covered by the output image.

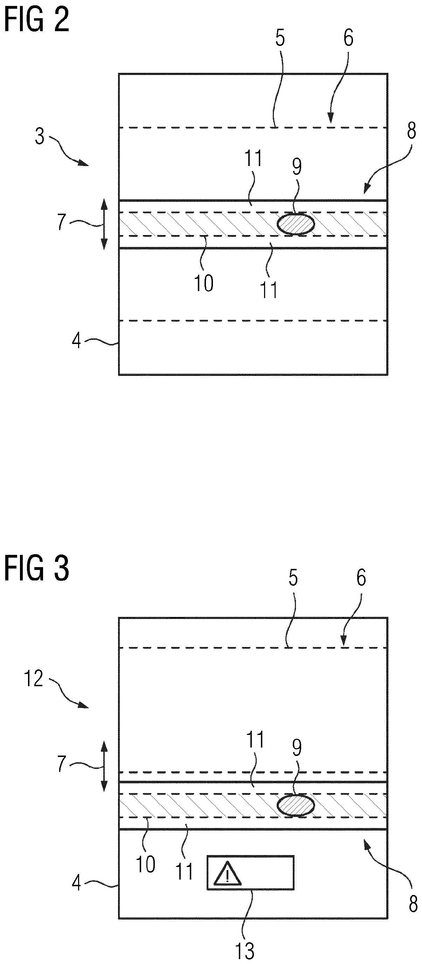

Fig. 2 shows a first example of a potential visualization. The output image 3 covers a display region 4 which is larger than, but comprises the acquisition region 5 of the included preparation image 6 in one direction, in this case the axial direction 7 (corresponding to the superior-inferior direction of the patient). In this case, the feature of interest is located within the acquisition region 5, such that its visual representation 8 comprising multiple representation elements is overlaid over the preparation image 6. The representation elements comprise the feature of interest 9 itself and an indicator 10 of its axial dimension. Furthermore, a visualization element 11 indicates a safety margin in axial direction 7. In this case the safety margin is chosen to account for potential misalignments due to breathing motion, which is often relevant in the thorax and abdomen region. Expected breathing motion amplitudes may be taken from a database of empirically determined values, in particular associated with body regions and/or anatomical structures, and/or may be determined as a patient-specific estimate based on patient-specific prior information, which may, for example, be comprised in the source data set and/or at least one other prior imaging result data set. Other possible errors may be included, for example, registration errors provided by a registration function performing the registration in step S3.

The visual representation 8 allows a user to select appropriate acquisition parameters for the examination image, in particular regarding its imaged and/or reconstructed field of view. Optionally, at least a part of such acquisition parameters may be automatically determined and confirmed by the user. For example, a proposed field of view of the examination image may additionally be shown in the user interface, in particular in the output image 3.

Fig. 3 shows an example of an output image 12, in which the feature of interest 9 lies outside of the acquisition region 5 of the topogram, but inside the (here correspondingly larger chosen) display region 4. As a warning 13, a notice is displayed that informs the user about this circumstance. The user may then consider acquiring another topogram to include that feature of interest 9 in the acquisition region. However, the visual representation 8 of the feature of interest 9 is still shown at the correct position in the display region 4, in particular also indicating the axial range (indicator 10) occupied by the feature of interest 9 and the safety margins (visualization elements 11), to facilitate visual/manual adjustment of the acquisition region 5 and/or the field of view of the examination image. In particular, an additional warning or notice can inform the user whether the feature of interest 9 is reliably included in the field of view of the examination image according to the current acquisition parameters.

In later steps not shown here, final acquisition parameters for the main scan of the examination image may be chosen/confirmed using the user interface, and the examination image may be acquired and analyzed, in particular regarding the feature of interest 9.

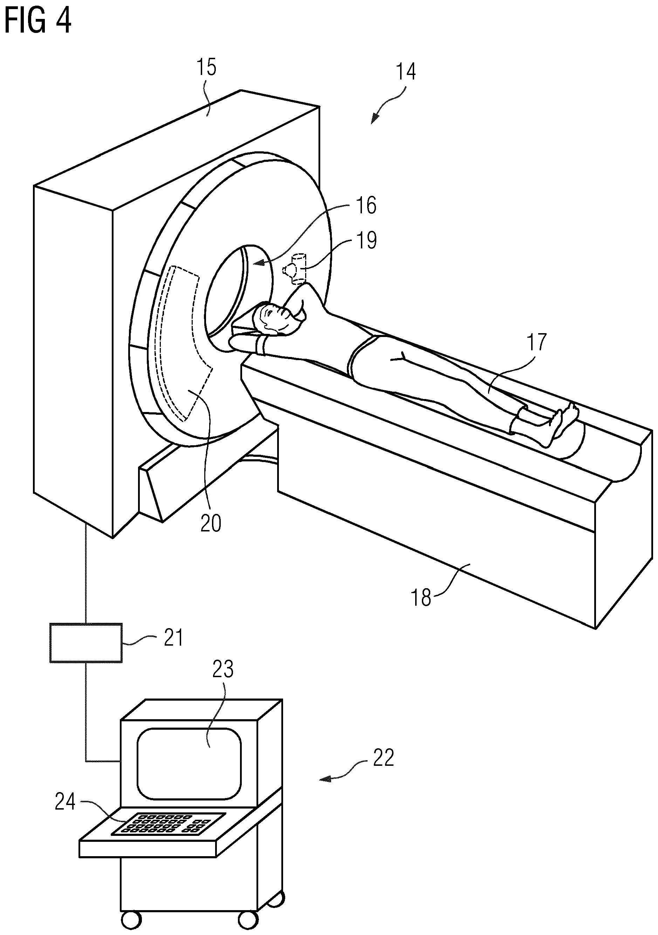

Fig. 4 shows a principle drawing of a medical imaging device 14 according to the invention, in this case a computed tomography device. The medical imaging device 14 comprises a gantry 15 having a patient opening 16, into which a patient 17 can be introduced using patient table 18. In the gantry 15, a rotatable acquisition arrangement comprising an x-ray source 19 and an x-ray detector 20 is placed.

Operation of the medical imaging device 14 is controlled by a control device 21, which is also configured to execute a method as described regarding Fig. 1. The control device 21 is connected to a user interface device 22, comprising a display device 23 and an input device 24.

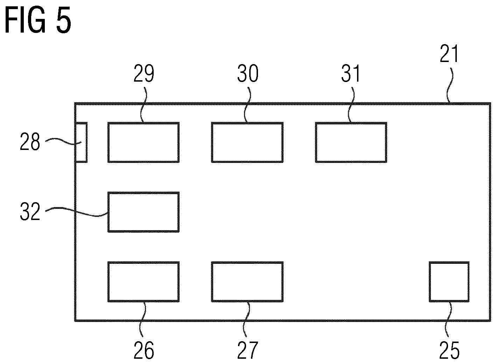

Fig. 5 shows the functional structure of the control device 21. The control device 21 comprises a storage means 25 for storing information, for example the preparation image 6, the retrieved source data set and the like. An acquisition unit 26 controls the acquisition arrangement to acquire image data, in particular also the preparation image 6 in step S1 and the acquisition of the examination image later on. A reconstruction unit 27 may be associated with the acquisition unit 26 to reconstruct three-dimensional images from acquire two-dimensional projections, as in principle known in the art.

Via a storage interface 28, the control unit is connected to the storage 2 for retrieving the source data set selected from the prior imaging result data sets 1 according to step S2 by a selection unit 29. The control device further comprises a registration unit 30 for determining the transformation according to step S3 and a compilation unit 31 for determining the output image 3, 12 according to step S4.

An output unit 32, which generally provides the user interface outputs the output image 3, 12 on the display device 23.

Independent of the grammatical term usage, individuals with male, female or other gender identities are included within the term.

## Claims

1. Computer-implemented method for operating a medical imaging device (14), wherein, for preparing an examination scan of a subject, a preparation image (6) of an acquisition region (5) is acquired, the preparation image (6) being defined with respect to a first coordinate system and used to define at least one parameter for acquiring an examination image, comprising the steps of
- retrieving, from a storage (2) comprising at least one prior imaging result data set (1) of the subject, a source data set chosen from the at least one prior imaging result data set (1), each prior imaging result data set (1) comprising a prior image showing at least a part of the acquisition region (5) and annotation information indicating at least one feature of interest (9) of the subject, wherein the annotation information comprises a location information describing a location of a respective feature of interest (9) in a second coordinate system of the prior image,
- registering the prior image of the source data set and the preparation image (6) to obtain registration information comprising a transformation from the second coordinate system to the first coordinate system,
- compiling an output image (3, 12) comprising the preparation image (6) and at least one visual representation (8), which is positioned correctly according to the location information of the source data set for at least one direction, of all features of interest (9) within a display region (4) comprising the acquisition region (5), and
- outputting the output image (3, 12) at a display device.

2. Method according to claim 1, **characterized in that** the examination scan is a follow-up examination scan, wherein the at least one prior imaging result data set (1) relates to a previous examination scan and/or is a baseline scan and/or wherein the examination scan is performed for comparison with the at least one prior imaging result data set (1).

3. Method according to claim 1 or 2, **characterized in that** the medical imaging device (14) is a computed tomography device and the preparation image (6) is a topogram.

4. Method according to one of the preceding claims, **characterized in that** the source data set is chosen from the multiple prior imaging result data sets (1) using at least one selection function, wherein the selection function evaluates at least one selection criterion.

5. Method according to claim 4, **characterized in that** at least one of the at least one selection criterion is chosen from the group comprising
- the acquisition date of the prior imaging result data set (1), wherein newer prior imaging result data sets (1) are preferred,
- the body region the prior imaging result data set (1) relates to, wherein prior imaging result data sets (1) relating to the same body region as the examination scan are preferred,
- the modality the prior imaging result data set (1) has been acquired with, wherein prior imaging result data sets (1) being acquired with the same modality as the one used for the examination scan are preferred,
- motion state information associated with the prior image, wherein prior images having a similar motion state to the preparation image (6) are preferred, and
- acquisition protocol parameters, wherein prior imaging result data set (1) using acquisition protocols similar to that of the acquisition scan are preferred.

6. Method according to one of the preceding claims, **characterized in that** the preparation image (6) is two-dimensional and the prior image is at least three-dimensional, such that registration is performed as 2D-3D-registration.

7. Method according to claim 6, **characterized in that** the 2D-3D-registration comprises at least one of
- landmark-based registration, and
- minimizing a difference metric between preparation image (6) and a two-dimensional reference image determined, in particular by forward projection, from the prior image, in particular based on an assumed acquisition geometry of the preparation image (6) in the second coordinate system.

8. Method according to one of the preceding claims, **characterized in that** the preparation image (6) is a two-dimensional projection image and the annotation information comprises a three-dimensional location information, wherein, during compilation of the output image (3, 12), the three-dimensional location information is forward projected into the projection geometry of the preparation image (6) according to the transformation to facilitate correct positioning of the respective feature in the output image (3, 12).

9. Method according to one of the preceding claims, **characterized in that**, if a feature lies outside the acquisition region (5) according to the transformation, a warning (13) is output to a user and/or the feature of interest (9) is displayed at the correct position according to the location information outside the preparation image (6) in the display region (4).

10. Method according to claim 9, **characterized in that** at least one acquisition parameter for the examination image, which describes the field of view of the examination image, in particular an axial examination range, is adapted such that the feature of interest (9) lying outside the acquisition region (5) is included in the field of view.

11. Method according to one of the preceding claims, **characterized in that** an expected location error in at least one direction, in particular the superior-inferior direction, due to breathing and/or registration is determined and visualized in the output image (3, 12) by at least one visualization element (11).

12. Method according to one of the preceding claims, **characterized in that** the annotation information comprises at least one chosen from the group of
- DICOM meta information,
- a reference to a partial image of the or a prior image, in particular to a slice,
- at least one extension of the feature, in particular a lesion diameter,
- a textual entry describing the position of the feature in at least one of the at least one direction, and
- an, in particular three-dimensional, segmentation result of the feature.

13. Medical imaging device (14), in particular computed tomography device, for an examination scan of a subject, comprising an acquisition arrangement, a display device and a control device, wherein the control device comprises:
- an acquisition unit for controlling the acquisition arrangement to acquire a preparation image (6) of an acquisition region (5), the preparation image (6) being defined with respect to a first coordinate system and used to define at least one parameter for acquiring an examination image, and the examination image,
- a storage interface for retrieving, from a storage comprising at least one prior imaging result data set (1) of the subject, a source data set chosen from the at least one prior imaging result data set (1), each prior imaging result data set (1) comprising a prior image showing at least a part of the acquisition region (5) and annotation information indicating at least one feature of interest (9) of the subject, wherein the annotation information comprises a location information describing a location of a respective feature of interest (9) in a second coordinate system of the prior image,
- a registration unit for registering the prior image of the source data set and the preparation image (6) to obtain registration information comprising a transformation from the second coordinate system to the first coordinate system,
- a compilation unit for compiling an output image (3, 12) comprising the preparation image (6) and at least one visual representation (8), which is positioned correctly according to the location information of the source data set for at least one direction, of all features of interest (9) within a display region (4) comprising the acquisition region (5), and
- an output unit for outputting the output image (3, 12) at the display device.

14. Computer program, which, when it is executed on a control device of a medical imaging device (14), causes the control device to perform the steps of a method according to any of the claims 1 to 12.

15. Electronically readable storage medium, on which a computer program according to claim 14 is stored.
